# EUROPEAN PATENT SPECIFICATION

(11) **EP 2 386 268 B1**
(45) Date of publication and mention of the grant of the patent: **08.11.2017**
(21) Application number: 11006532.3
(22) Date of filing: 20.12.2005
(51) Int. Cl.: A61F 2/00

(54) **Treatment of anal incontinence**
Behandlung analer Inkontinenz
Traitement de l'incontinence anale

(30) Priority: 22.04.2005 US 673878 P; 20.12.2004 US 637665 P
(43) Date of publication of application: 16.11.2011
(62) Divisional of application: 05854848.8
(73) Proprietor: Rosenblatt Associates, LLC, Newton, MA 02465 (US)
(72) Inventor: Rosenblatt, Peter L., Newton, Massachusetts 02465 (US)
(74) Representative: Vossius & Partner Patentanwälte Rechtsanwälte mbB

(56) References cited:
- FR-A1- 2 852 817
- US-A- 3 789 828
- US-A- 4 786 276
- US-A1- 2004 039 453

## Description

### BACKGROUND

Anal incontinence is a common problem that occurs in both men and women, though is certainly more prevalent in women after vaginal childbirth, presumably the result of trauma to pelvic floor muscles, supporting fascia and nerves. Fecal incontinence affects an estimated 7.6 percent of women between the ages of 30 - 90. The prevalence increases with age, affecting 3.6 percent of women between 30 - 39 and 15.2 percent of women between 80 - 90. Several factors contribute to anal continence, including the resting tone of the external and internal anal sphincters, as well as the position of the levator ani muscles, especially the puborectalis muscle, which forms a sling around the rectum and is responsible for the so-called "ano-rectal angle," which keeps stool in the rectum until voluntary defecation relaxes the puborectalis muscle and straightens the angle, allowing stool to move towards the anus.

Defecation is often aided by expulsive abdominal forces. Anal incontinence may occur as the result of several mechanisms, including direct damage to the internal or external anal sphincters (from iatrogenic episiotomy or spontaneous lacerations during vaginal delivery), or to the levator ani muscles. It may also result from indirect injury of these muscles through denervation of the nerves that supply these muscles. Treatment of this problem has centered on pelvic floor rehabilitation, dietary changes, or surgical correction. Surgery has been used to treat specific defects in the anal sphincters, such as external anal sphincteroplasty. Success rates of only 50% or less are generally reported for these procedures on long-term follow-up.

More recently, an artificial anal sphincter has been used to bypass these muscles, though this surgery involves fairly extensive dissection and requires the patient to depress a subcutaneous valve which temporarily deflates the sphincter cuff and allows voluntary
defecation. This procedure is performed in very few centers in the U.S., and even in experienced hands, complications occur frequently. Dynamic graciloplasty, which involves mobilization and wrapping of the gracilis muscle around the anorectum is now another accepted procedure although is remains complex and requires extensive experience to obtain good results. More recently, sacral nerve stimulation has been used with some success to treat fecal incontinence, though the mechanism of success in these patients remains unclear, and may not be appropriate in women with obvious anatomic abnormalities, such as anal sphincter or levator muscle disruptions.

In addition, many women report other symptoms of bowel dysfunction, such as constipation and incomplete bowel emptying. For some women, these symptoms are due to either a anterior rectocele (a hernia of the rectum into the vaginal canal), or due to a defect in the levator ani muscles, which results in descent of the levator plate and / or perineum with abdominal straining. In addition, patients may be noted to have a defect in the posterior aspect of the rectum, or a posterior rectocele. There are very few treatment options for this condition, though retrorectal levatorplasty has been used in the past. In this procedure, an incision is made between the anus and the coccyx and the levator muscles are exposed bilaterally. Sutures are then placed in the levator muscles to plicate them together in the midline.
Active (inflatable) and passive Male and Female urethral prosthesis are known in the art, for example from US patent application 2004/0215054 (active) and US patent 3,789,828 (passive). These are surgically inserted into the patient to apply pressure to the urethra when normal muscular stoppage of urinary flow is not functional. US2004/0215054 discloses an inflatable pressure pad that can be inflated to block flow or deflated to allow flow and US 3,789,828 discloses a device that permanently applies resilient pressure to the urethra to block it. Flow is effected either by applying pressure via the bladder muscles of by manually pressing on the bladder to overcome the resilient pressure. US Patent Application 2004/0039453 discloses an implant for treating pelvic floor disorders having a central support are and attachment arms extending therefrom.

### SUMMARY

According to the present invention, there is provided a system according to claims 1 to 20.

US patent no. US 4,786,276 discloses a sphincteric web for the treatment of incontinence having a particular alignment of three inflatable pressure cushions by which to prosthetically replicate the geometry of a normally continent rectum. The sphincteric web surrounds and embraces a patient's colon (or rectum).

### BRIEF DESCRIPTION OF THE DRAWINGS

FIG. 1 depicts the anatomy of the bony pelvis.
FIG. 2 depicts an exemplary placement of a device in the pelvis.
FIGS. 3-4 depict steps in one exemplary placement method.
FIGS. 5-6 depict steps in another exemplary placement method.
FIG. 7 depicts an exemplary final position of a length of supporting material.
FIGS. 8-10 depict exemplary uses of two lengths of supporting material.
FIGS. 11-19 depict exemplary uses of various instruments to position a length of supporting material.
FIGS. 20-22 depict exemplary slings.
FIGS. 23-25 depict exemplary slings having fluid-filled sacs.
FIGS. 26-29 depict an exemplary use of a stylet having a loop.
FIGS. 30-31 depict an exemplary use of a stylet having a hook.
FIGS. 32-38 depict additional exemplary embodiments of slings.
FIG. 39 depicts use of an exemplary device to measure the ano-rectal angle.
FIGS. 40-41 depict placement of a sling having a saddle-shaped central portion.

### DETAILED DESCRIPTION

The present disclosure provides a variety of systems and methods for treating anal incontinence. The normal ano-rectal angle can be restored by inserting a disclosed device under the posterior rectum, which may be supported with a synthetic or natural material in a sling-like position behind the anus and/or rectum. A posterior supporting apparatus may provide partial or complete closure of the rectum and/or anus with the posterior supporting apparatus.

In one embodiment, a synthetic or natural sling material may be placed under the rectum and may be supported by its arms, which may be extended up in a sling-like fashion through the obturator foramen bilaterally, or retropubically to the suprapubic region. In another embodiment, the device placed posterior to the rectum may include an inflatable or fluid-filled sac, which may or may not be adjusted post-operatively, by either changing its position or by altering the amount of fluid material, for example saline or a hydrogel, within the sac. "Fluid" is understood to include gasses, liquids, and semisolid media (such as gels). The central portion of the sling has a curved shape, such as a saddle shape, to help it conform to the external contour of the anus, rectum, ano-rectal angle, and/or levator ani muscles.

In a method of treating anal incontinence using the invention, an incision may be made between the anus and the coccyx and dissection performed, whereby the levator muscles and the levator plate are exposed. A small nick may be made on the medial thigh just lateral to the ischiopubic ramus and an introducer needle may be placed through the medial thigh incision, around the ischiopubic ramus, and be directed posteriorly into the ischiorectal fossa. The needle may then be directed lateral to the levator muscles, optionally with the assistance of a surgeon palpating the instrument though the vagina. The needle may then be brought posterior to the rectum, exiting the incision that was made in the midline. Alternatively, the needle may be passed from the midline incision between the anus and coccyx to the medial thigh incision lateral to the ischiopubic ramis. In an example, a suture may be threaded onto the eye of the needle, which in this case may be placed from the medial thigh to the incision between the anus and the coccyx, and may then be withdrawn through the tissue and held on the medial thigh. The procedure is repeated on the contralateral side. A synthetic (i.e., polypropylene, polyester, etc.) mesh (such as tension-free vaginal tape, TVT) or natural graft material may then be attached to each of the sutures coming from the midline incision, and the mesh may then be brought up through the medial thigh incisions by pulling up on the sutures.

The mesh may have a covering plastic sheath, which can facilitate passage through the tissues. The sheath may be removed when the sling is properly adjusted.

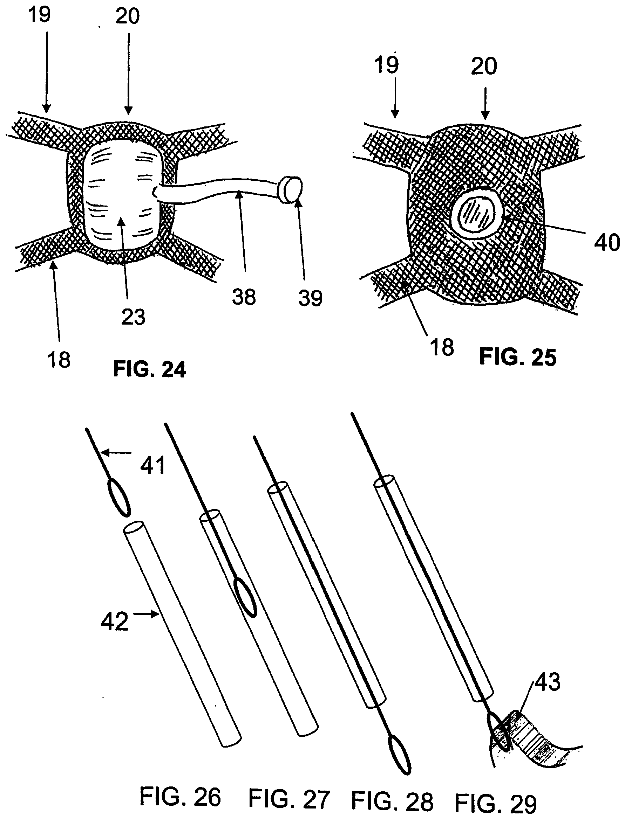

In another method, the needle that is passed through the tissue may have a hollow sleeve or tube over it (e.g., made of plastic, metal, or the like), and after passage, the needle may be withdrawn through the tissue, leaving the hollow sleeve in place. A stylet (e.g., made of plastic, metal, or the like) may then be then placed in the tube. The stylet may have a connector, such as a hook or a loop, so that a length of supporting material, such as a synthetic mesh (e.g., made of polypropylene or the like) or a natural graft, may be attached to the stylet connector. Exemplary uses of stylets with hooks or loops are shown in FIGS. 26-31. Once this procedure is performed bilaterally, the supporting material may be positioned under the rectum and the tension on the arms of the sling are adjusted. If the sleeves or tubes are utilized, the mesh can be adjusted before withdrawing the sleeves or tubes.

In another method, two passes of the needles can be made on each side, one approximately at the level of the medial superior portion of the obturator foramen, and the other several centimeters inferior and slightly more lateral (at the inferior portion of the obturator foramen). This permits two lengths of supporting material (also called "mesh" but not necessarily limited to mesh) to be brought up on each side. These mesh strips are attached to a central mesh that may be placed under the rectum, which may be a pre-formed mesh, or may be constructed by attaching the central mesh to the four mesh strips - two on each side. The subrectal portion may be synthetic mesh, or may be made of another material, such as an inflatable or fluid-filled polymer sac. The sub-rectal element provides
support to the posterior anus and/or rectum, and creates an angle between the anus and rectum, which keeps stool in the rectum until voluntary defecation.

In another method of using the invention, after the needle passes through the tissue, and is withdrawn, leaving a hollow tube in place, a plastic or metal stylet, previously fixed to the mesh with or without a sheath, can be placed up from the sub-rectal incision to the medial thigh incision and can be held. The mesh may then be brought up through the tubing by pulling on the stylet from above. Once the sling end comes out from the tubing, the hollow tube can be removed, after the sling has been adjusted for proper tensioning.

In another method of using the invention, the posterior aspect of the sub-rectal portion may be attached to the coccyx by one of several methods, such as direct suturing or with bone anchors. Such attachment can help maintain the position of the sub-rectal portion, which, in effect, restores the structure and function of the levator plate. Alternatively, the sub-rectal portion may have an extension coming off its inferior portion, which extends out and is fixed to the coccyx.

The fluid-filled sac under or adjacent to the rectum may have a port, such as a subcutaneous port, that may allow for fluid addition or removal in the post-operative period. This port may facilitate post-operative adjustment of the size and / or shape of the sac to provide for optimal results. The subcutaneous port may be placed directly under the sac, in the perineal skin, or may be connected to the sac by means of a connector tubing so that the port does not need to be located in the perineum itself, but instead may be positioned in a number of areas, including, for example, the buttocks.

The needle may have a hook near the end, that can be covered during insertion, but that may be exposed after the needle has been placed through the tissue. The user implanting the device may operate a switch or other actuator, such as a spring-loaded mechanism, to expose the hook. The arm of the sling, or a pre-loaded suture on the sling- arm, may then be placed on the hook and the needle withdrawn through the tissue.

In another method of using the invention, once the needle is placed through the tissue, the tip of the needle may be unscrewed off the end of the needle shaft. The arms of the sling may have a device attached to each end that may screw onto the needle shaft or otherwise fasten onto the needle shaft and then the needle is withdrawn, bringing the sling arm through the tissue.

A sheath covering the needle may remain in place in order to facilitate the movement of the synthetic material through the tissue, which is only removed once the tension on the sling is adjusted. The sheath may be deformable, rather than rigid or semi-rigid, and may be flattened after removal of the needle, to accommodate the flat shape of the sling material itself.

The needle could have blunt metallic insert (to maintain the strength of the needle) with a plastic covering sheath that has a sharp needle tip configuration on the end. After the needle is placed through the tissue, the metallic blunt needle is withdrawn, and the plastic needle tip cut off. A suture retriever is then placed anterograde through the hollow plastic tube and grasps a suture that has been attached or pre-attached to the sling. The sling is withdrawn through the plastic tube and the tube is removed once the sling is adjusted.

In one embodiment, the needle tip may be made from two separate pieces that act as jaws that open to catch the mesh or suture attached to the mesh, after the needle is passed through the tissue from the medial thigh to the incision posterior to the anus. This needle may be introduced with a plastic outer covering, so that the sling material may be drawn up through the tissue without catching on the surrounding structures. Once in proper position, the surgeon may remove the plastic sheath, which would then allow for the synthetic mesh to become fixed in the tissues.

A curved metal needle may be placed through the tissue from the medial thigh to the perineal incision. The end of the needle may be unscrewed, and the sling with attached plastic or metal piece may be screwed or snapped onto or into the connector on the needle. The sling, possibly with covering sheath, may then be withdrawn through the tissue and held and the plastic sheath is removed after the sling has been adjusted.

The shape of the sling may be a fixed width throughout its length. Alternatively, the central portion that is positioned under/behind the rectum may be wider than the arms. The central portion is curved to help it conform to the shape of the tissue it is supporting. The curved shape may be a saddle shape, such as roughly a hyperbolic paraboloid or resembling a PRTNGLES(R) brand potato crisp. The central portion is preformed with the curved shape.

The mesh may be continuous throughout the length of the sling, or may have a central portion that includes a fluid-filled sac that is affixed to the sling anns on the sides. Preferably, the synthetic mesh would be continuous throughout its length in order to provide a backboard of support under the rectum and under the fluid-filled sac, if the fluid- filled sac is employed.

The fluid sac may have a circular or elongated shape under the rectum, or may include several compartments that can be separately filled with several access ports, in order to change the occlusion of the rectum. The fluid filled sac may have the curved shapes as discussed above.

Wings may connect a sling central portion to the arms of the sling. The wings may be made of mesh or other supporting material.

In another embodiment, the sling may be a hybrid of materials, comprised of, for instance, a polypropylene mesh along the arms of the sling in order to have self-attaching properties to the obturator fascia, and a natural xenograft material, such as porcine small intestinal submucosa, or an allograft, such as cadaveric fascia, located under and or lateral to the anus / rectum.

In another embodiment, the arms of the sling may include a synthetic material such as silastic or other plastic, and may have serrations that grab on to the obturator fascia as the arms are pulled through the tissue.

In another embodiment, the arms of the sling include sutures. There may be several sets of sutures on each side, in order to prevent the sub-rectal portion of the sling from rolling up underneath the anorectum.

In another embodiment, the arms of the sling may be attached to pelvic bone, such as the inferior-medial portion of the ischiopubic rami, or the inferior portion of the pubis, with bone anchors, suture material, or other fixation devices.

In another embodiment, the material under and / or lateral to the ano-rectum includes a synthetic material, such as silastic or other plastic material, that may be flexible, to conform to the shape of the bowel.

In another embodiment, a number of synthetic or natural elements are attached to the mesh in a direction transverse to the length of the sling, such as perpendicular or substantially perpendicular to the length of the sling. The elements may be semi-rigid and may be so positioned in the mesh as to be located under or lateral to the bowel when the mesh is deployed, for the purpose of keeping the mesh from rolling up underneath the anorectum. For example, the graft may have a stiff or flexible bar incorporated into the graft, located on either side of the rectum, to prevent rolling of the graft material.

In another embodiment, the sling may have additional straps attached to the subrectal portion that penetrate posteriorly, such as on either side of the coccyx, that may pass through the subcutaneous tissue and hold the graft in position, to prevent rolling of the subrectal mesh.

In another embodiment, the synthetic material may be elastic, which may permit stretching of the sling with abdominal straining, such as occurs with voluntary defecation.

In use, the sling may be passed through the levator ani muscle, rather than behind the muscles.

In another embodiment, not forming part of the claimed invention, the system may include a device used to evaluate the anorectal angle, for pre-operative diagnosis, intra-operative adjustment, and/or post-operative evaluation. The device is sufficiently flexible that it can be flexed to conform to the anorectal angle. The amount of flexion may be measured, thereby establishing the shape of the ano-rectal angle. In one example, the device may be inserted into the rectum and has a flexible joint which is placed at the junction between the anus and rectum. The device may then measure the angle created between the rectal and anal portions, and this angle may be displayed visually on the device, in one of a number of manners, including a dial or a digital display. The angle may also be communicated to an external display for convenience. The device may include a rotation- or position-sensitive transducer. This ano-rectal angle measurement device may be adapted so that is fits over the examiner's gloved finger with a portion that fits over or on the examiner's distal finger, and another portion that fits over or on the proximal finger. In this manner, when the examiner bends his or her finger to determine the ano-rectal angle, the measured angle is recorded visually on a display.

Various portions of the device may be coated, impregnated, or formed with one or more drugs to be eluted to an adjacent tissue. Various portions of the device may be formed of biodegradable or bioabsorbable material.

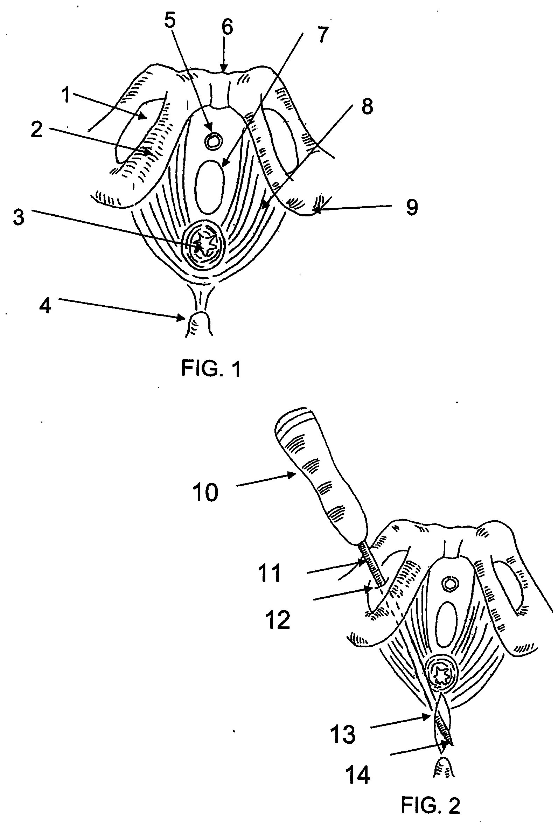

FIG. 1 shows the anatomy of the bony pelvis, with the pubic symphysis (6), the ischiopubic ramus (2), the ischial tuberosity (9), the coccyx (4), and the obturator foramen (I)- It also demonstrates the relationship of the levator ani muscles (and, in particular, the puborectalis (8)) to the urethra (5), vagina (7), and rectum (3).

FIG. 2 demonstrates the placement of a needle (11) and attached handle (10) from the medial thigh incision (12), through the obturator membrane, into the ischiorectal fossa, and the needle tip (14) emerging through the vertical incision (13) between the anus and the coccyx.

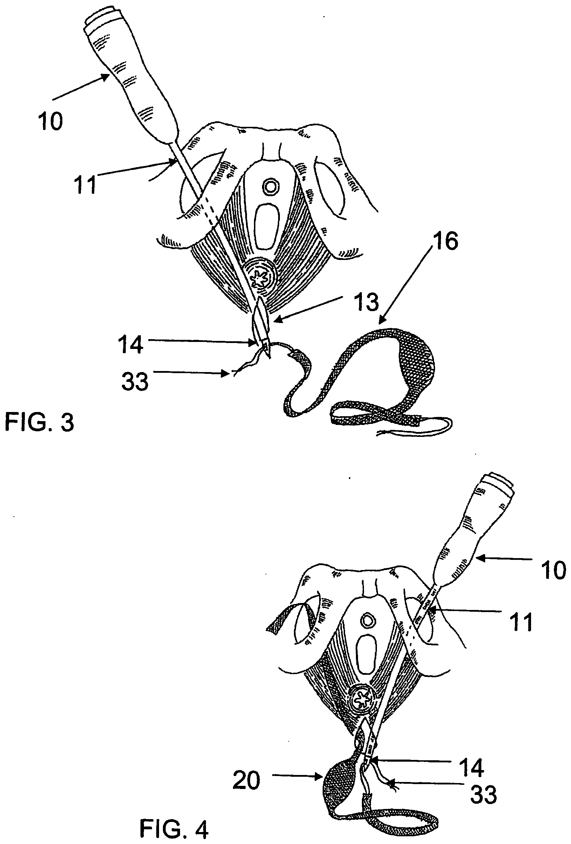

FIGS. 3-4 depict steps in one exemplary placement method. In FIG. 3, the needle (11) with the attached handle (10) has been placed through the thigh incision and through the obturator foramen, through the ischio-rectal fossa and out through the incision (13)
between the anus and the coccyx. A suture loop (33) attached to the sling (16) is grasped by the needle tip (14) in order to transfer the sling to the thigh incision. FIG. 4 demonstrates the second pass of the needle on the contralateral side with the handle (10) and needle (11) in place. The tip (14) is grasping the suture loop (33) in order to pull the other arm of the sling up through to the thigh incision. This allows the central portion of the sling (20) to rest under the ano-rectal area.

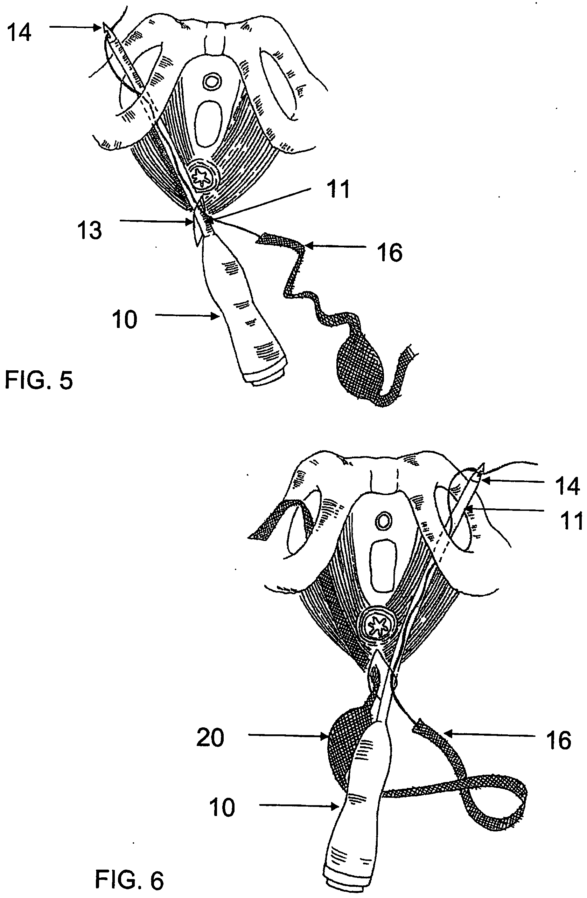

FIGS. 5-6 depict steps in another exemplary placement method. In FIG. 5, the needle (11) with attached handle (10) has been placed from the post-anal vertical incision (13) up through the ischiorectal fossa, through the obturator foramen, and out through the thigh incision, and has transferred a suture attached to the sling (18) to the thigh region. This allows the sling to be brought through the tissues up to the region of the thigh. FIG. 6 shows the right side of the sling in place and the needle (11) with attached handle (10) transferring a suture attached to the left side of the sling arm (16) up through the left side. The suture is being held by the needle tip (14).

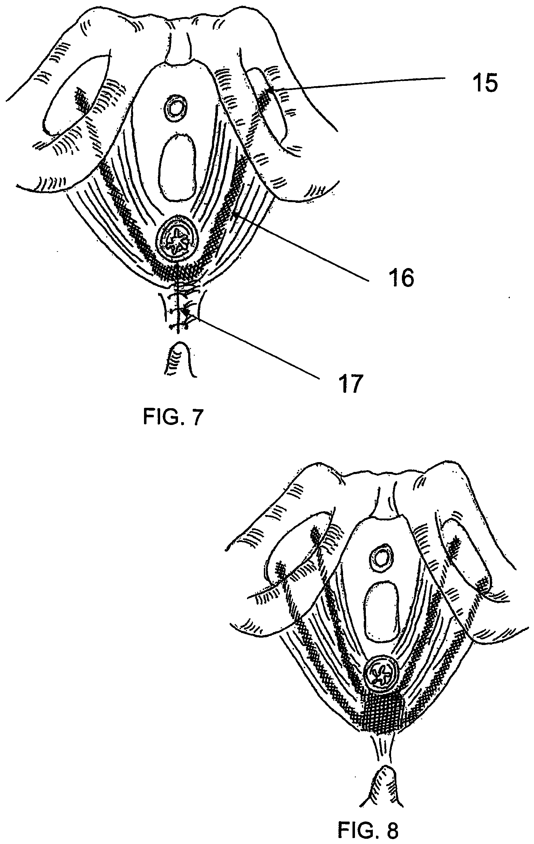

FIG. 7 demonstrates a final position of the synthetic mesh (16) under the anus and/or rectum, with the incision between the anus and the coccyx (17), and up through the medial portion of the obturator membrane (15).

FIG. 8 demonstrates the use of two synthetic mesh straps placed through the obturator membrane, the first more distal and placed near the superior-medial aspect of the obturator foramen (18), and the second placed near the inferior portion of the obturator foramen (19), and attached to a central element (20).

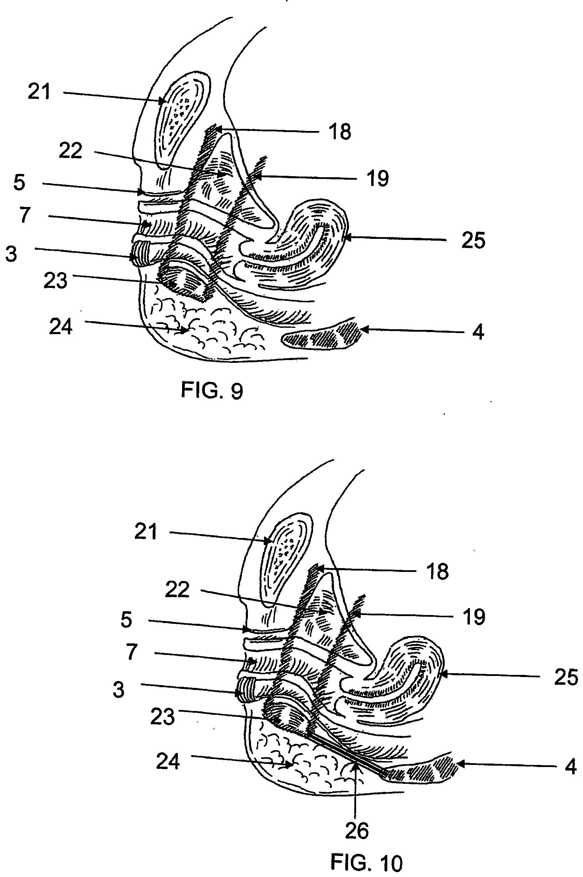

FIG. 9 shows a lateral orientation of the pelvis with the pubic symphysis (21), the bladder (22), the uterus (25), and the ischiorectal fossa (24) with two synthetic straps on each side, the first more distal (18) and the other more proximal (19), and a sub-rectal element (23) that includes a fluid or gas filled reservoir.

FIG. 10 shows a lateral orientation of the pelvis with the sling in place, with an extension (26) of the sub-rectal element attached to the coccyx with the use of sutures, bone anchor, or other method of affixing the synthetic material to the coccyx.

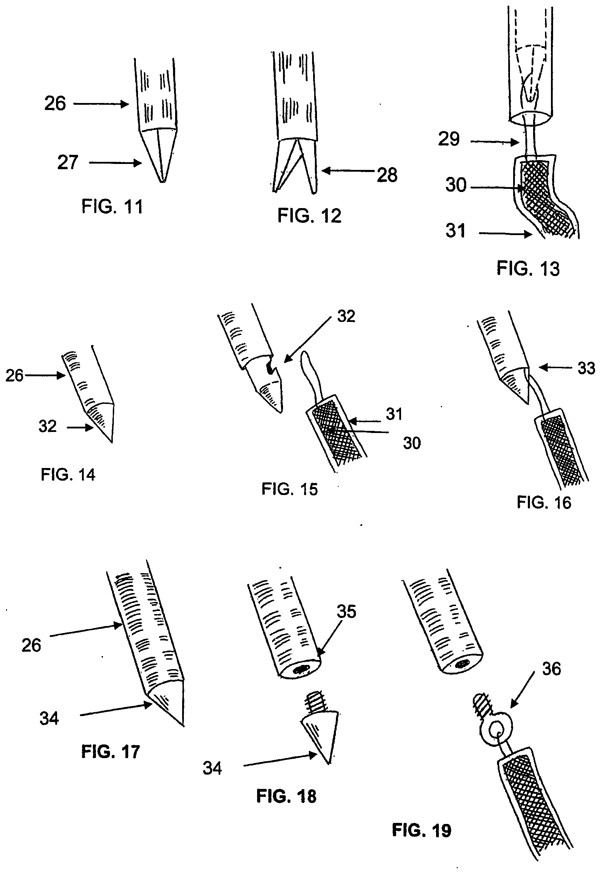

FIGS. 11-13 demonstrate the use of a needle (27) introducer placed from the medial thigh to the incision under of the rectum. Once through the tissue, the jaw opens in the middle, which reveals a grasping instrument (28) that can hold on to a suture (29) affixed to the mesh (30) with or without a plastic sheath (31). The sling material is then brought through the tissue, with or without a plastic outer tubing (26) through which the grasper had been placed during the needle insertion.

FIGS. 14-16 demonstrate a needle (32) that, after insertion through the tissue, can be advanced beyond the outer sheath, with or without a spring-mechanism to deploy the needle. This reveals a notch, on which the suture loop (previously attached to the mesh), can be placed (33), and the sling (30) is then brought up through the tissues to the medial thigh

FIGS. 17-19 demonstrate a needle (34) that, after insertion through the tissue, may be separated from the shaft of the needle by unscrewing the needle tip. The sling would have a male-connector screw (36) that attaches to the straight needle shaft (35) and then the needle is withdrawn, which draws the mesh up through the tissue.

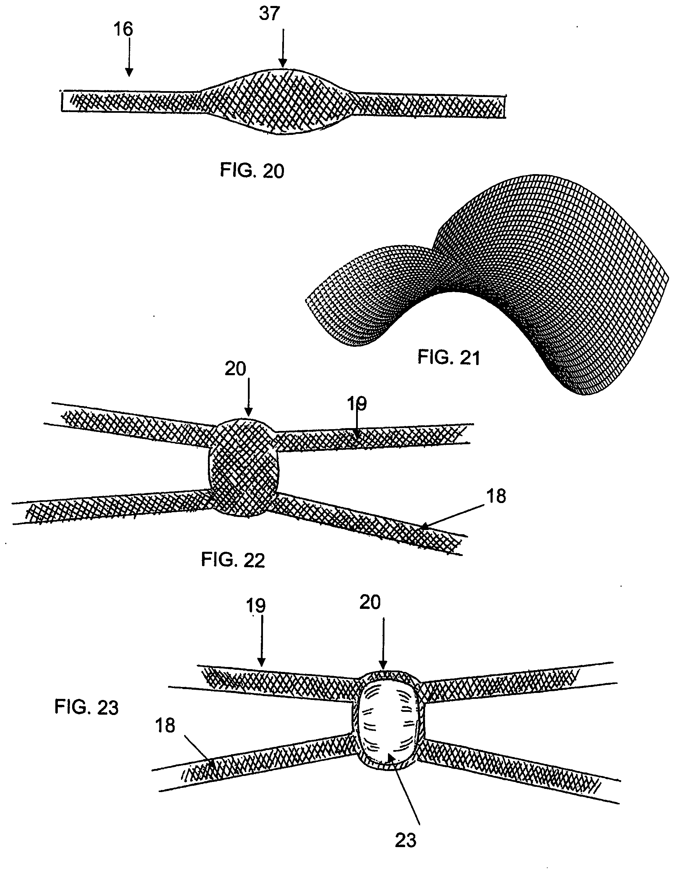

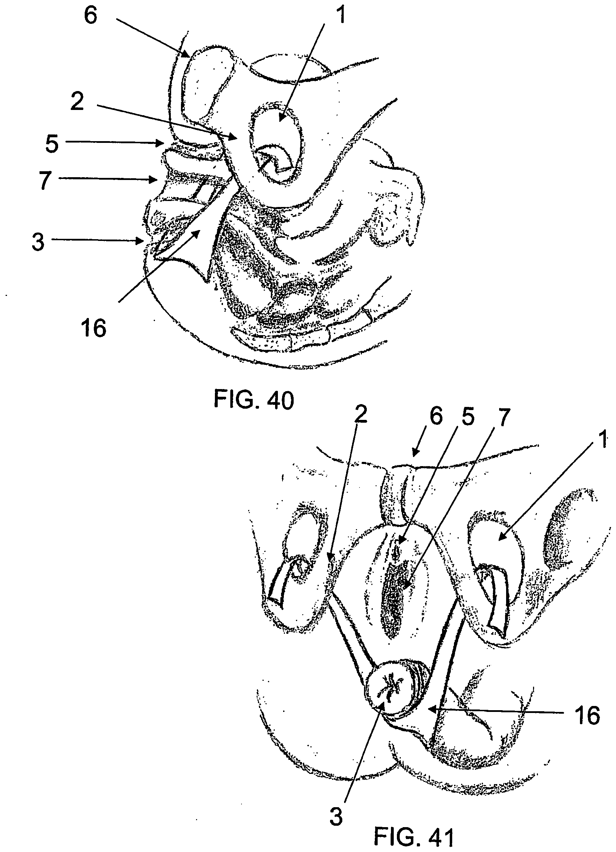

FIG. 20 demonstrates a sling that has narrow arms (16) and a wider area which would sit under the rectum (37) and distribute forces over a wide area. FIG. 21 illustrates a sling central portion (arms not shown) that has a curved shaped, specifically, a saddle shape. The saddle shape may facilitate making good contact with the anatomy to be supported. Its positioning is illustrated in FIGS. 40-41. One curve of the saddle allows the sling to arc between the obturator regions, while the other curve can complement the anorectal angle. FIG. 22 demonstrates an elongated central sling (20) with four attached arms, two of which are passed from the medial superior portion of the obturator membrane (18), and the other two which are passed through the inferior portion of the obturator membrane (19).

FIG. 23 demonstrates a superior view of a mesh that has a fluid-filled sac on the superior side of the graft material.

FIG. 24 shows another embodiment of a sling having a central portion with an inflatable sac. Connector tubing is attached to the fluid-filled sac and can be placed under the buttocks or other location within the reach of the tubing, and has a port at the end that can be used for filling or reducing the amount of fluid is contained within the sac.

FIG. 25 demonstrates an inferior view of the central portion (20), which shows a port from the fluid-filled sac coming out through a hole at the bottom of the graft (40). This port may be accessed subcutaneously in order to either add more fluid or remove fluid.

FIGS. 26-29 exhibit an exemplary use of a loop stylet. Stylet (41) may be advanced through tube (42). A length of sling material (43) may be threaded through the loop so that
the material catches in the loop. The stylet may then be withdrawn back through the tube to bring the end of the sling material to the desired position.

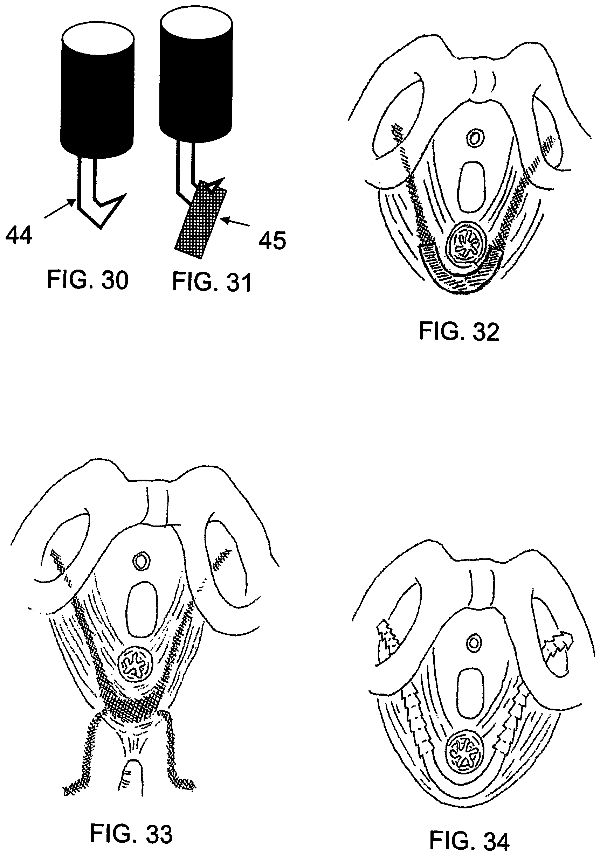

FIGS. 30-31 show an exemplary use of a hook stylet (44). A piece of sling material (45) may be stabbed onto a sharp tip of the hook. The hook may then be withdrawn through a tube to bring the end of the sling material to the desired location.

FIG. 32 shows a hybrid sling, comprised of, for instance, synthetic mesh arms attached to a central natural material placed under and / or lateral to the ano-rectum.

FIG. 33 shows the sling with additional straps of material that are attached to the subrectal portion of the device, and are secured in place by passage into subcutaneous tissue, in order to prevent rolling of the sling. The straps may be directed posteriorly, on either side of the coccyx, in order to keep the subrectal portion flat.

FIG. 34 demonstrates a sling made of a synthetic material such as silastic or other plastic with serrations on each arm that maintain the sling in position after adjustment by the surgeon

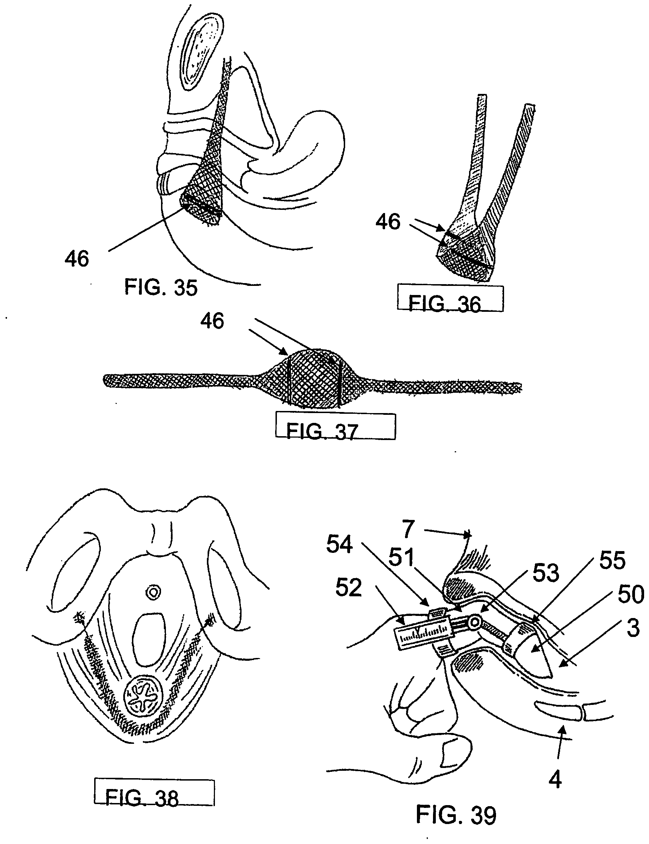

FIGS. 35-37 show embodiments of slings that include rigid or semi-rigid elements (46). The elements may be attached to the sling in order to keep the sling from rolling under the anorectal portion.

FIG. 38 shows the use of bone anchors to hold the sling into position, in this case to the inferior-medial portion of the ischiopubic rami.

FIG. 39 depicts a device, not forming part of the claimed invention, attached to the examiner's finger used to measure the angle between the rectum (3) and anus. The vagina (7) rests anterior to the anus and rectum, and the coccyx (4) is located posterior to the rectum. A proximal ring (54) is placed on the proximal phalanx (51) and the distal ring (55) is placed on the distal phalanx (50) and these are connected by a joint (53). The angle made between the anus and rectum is measured and displayed on a visual scale (52).

## Claims

1. A system for reducing anal incontinence including a rectal sling (16) implantable in a patient and an introducer needle (11) having a needle tip (14) and a needle shaft, said introducer needle (11) attachable to the rectal sling, the rectal sling having a central portion (20) attached to at least two arms (18, 19) extending therefrom **characterized by**: the central portion having a preformed curved shape that is wider than said at least two arms and which, after implantation, is structured to conform to the external contour of the anus, rectum, a no-rectal angle and/or levator ani muscles in said patient.

2. The system of claim 1 wherein said introducer needle (11) further comprises a hollow sheath (31) covering said introducer needle.

3. The system of claim 1 wherein said needle tip (14) is threadably removable from said needle shaft.

4. The system of claim 3 further including a male-connector screw (36) having a threaded end and a non-threaded end, said non-threaded end coupled to said arms (18,19).

5. The system of claim 4 wherein said male-connector screw (36) is structured to threadably couple to said needle shaft upon removal of said needle tip.

6. The system of claim 1 wherein said needle tip comprises a jaw for grasping said arms.

7. The system of claim 2 wherein said arms (18, 19) further include a suture coupled thereto.

8. The system of claim 7 wherein said sheath includes a spring-mechanism for deploying the needle tip (14).

9. The system of claim 7 or 8 wherein said needle tip (14) includes a notch for operably coupling to said suture.

10. The system of claim 1 wherein the central portion (20) includes an adjustably inflatable sac.

11. The system of claim 10 wherein said adjustably inflatable sac further includes a subcutaneously accessible port positioned at a bottom of the central portion for the introduction and removal of fluid.

12. The system of claim 10 wherein said adjustably inflatable sac further includes connector tubing (42) having first and second ends, said connector tubing coupled to said sac at said first end and having a port coupled thereto at said second end for introducing fluid into and removing fluid from said sac.

13. The system of claim 1 wherein said arms (18, 19) include serrations thereon for maintaining said sling (16) in position after implantation.

14. The system of claim 1 further comprising fixation devices for attaching said arms (18, 19) to pelvic bone, said fixation devices selected from the group consisting of bone anchors, suture material and combinations thereof.

15. The system of claim 1 wherein said central portion (20) further comprises rigid, semi-rigid or flexible elements coupled to said central portion in a direction transverse to the length of the sling (16).

16. The system of claim 1 further comprising a measuring device for measuring the angle made between the anus and rectum (37), said measuring device having a proximal ring and a distal ring operably connected by joint wherein said angle is displayed on a visual scale coupled to said proximal ring.

17. The system of claim 16 wherein said measuring device is structured to fit over a finger.

18. The system of claim 1 wherein each arm includes a removable sheath for facilitating passage through an obturator foramen.

19. The system of claim 1 wherein the sling (16) comprises a material selected from the group consisting of a synthetic material, a natural xenograft material, an allograft material and combinations thereof.

20. The system of claim 18 wherein each arm (18, 19) of the sling (16) is made from elastic material.

## Patentansprüche

1. System zum Verringern einer analen Inkontinenz, aufweisend eine in einen Patienten implantierbare rektale Schlinge (16) und eine Einführungsnadel (11) mit einer Nadelspitze (14) und einem Nadelschaft, wobei die Einführungsnadel (11) mit der rektalen Schlinge verbindbar ist, wobei die rektale Schlinge einen mittleren Abschnitt (20) hat, der an mindestens zwei Armen (18, 19) befestigt ist, die sich von dort aus erstrecken, **dadurch gekennzeichnet, dass** der mittlere Abschnitt eine vorgeformte gebogene Gestalt hat, die breiter als die mindestens zwei Arme ist, und der mittlere Abschnitt strukturiert ist, nach einer Implantation sich an die externe Kontur des Anus, Rektums, eines ano-rektalen Winkels und/oder Levatorani-Muskels des Patienten anzupassen.

2. System nach Anspruch 1, wobei die Einführungsnadel (11) ferner eine die Einführungsnadel bedeckende hohle Hülse (31) aufweist.

3. System nach Anspruch 1, wobei die Nadelspitze (14) von dem Nadelschaft abschraubbar ist.

4. System nach Anspruch 3, ferner mit einer Außengewinde-Verbindungsschraube (36), die ein mit Gewinde versehenes Ende und ein gewindeloses Ende aufweist, wobei das gewindelose Ende mit den Armen (18, 19) verbunden ist.

5. System nach Anspruch 4, wobei die Außengewinde-Verbindungsschraube (36) strukturiert ist, nach Entfernen der Nadelspitze an den Nadelschaft geschraubt zu werden.

6. System nach Anspruch 1, wobei die Nadelspitze eine Klemmbacke zum Greifen der Arme aufweist.

7. System nach Anspruch 2, wobei die Arme (18, 19) ferner ein daran angebrachtes Nahtmaterial aufweisen.

8. System nach Anspruch 7, wobei die Hülse einen Federmechanismus zum Entfalten der Nadelspitze (14) aufweist.

9. System nach Anspruch 7 oder 8, wobei die Nadelspitze (14) eine Kerbe aufweist, um in Verwendung das Nahtmaterial daran zu koppeln.

10. System nach Anspruch 1, wobei der mittlere Abschnitt (20) einen anpassbar aufblasbaren Beutel aufweist.

11. System nach Anspruch 10, wobei der anpassbar aufblasbare Beutel ferner eine an der Unterseite des mittleren Abschnitts angeordnete subkutan zugängliche Öffnung zum Einführen und Entfernen von Fluid aufweist.

12. System nach Anspruch 10, wobei der anpassbar aufblasbare Beutel einen Verbindungsschlauch (42) mit einem ersten und zweiten Ende aufweist, wobei der Verbindungsschlauch an seinem ersten Ende mit dem Beutel gekoppelt ist und an seinem zweiten Ende mit einem Anschluss gekoppelt ist, um Fluid in den Beutel einzuführen und aus dem Beutel zu entfernen.

13. System nach Anspruch 1, wobei die Arme (18, 19) Einkerbungen aufweisen, um die Schlinge (16) nach ihrer Implantation in Position zu halten.

14. System nach Anspruch 1, ferner mit Befestigungsvorrichtungen zum Befestigen der Arme (18, 19) an einem Beckenknochen, wobei die Befestigungsvorrichtungen aus der folgenden Gruppe ausgewählt sind: Knochenanker, Nahtmaterial und Kombinationen davon.

15. System nach Anspruch 1, wobei der mittlere Abschnitt (20) ferner steife, halbsteife oder biegsame Elemente aufweist, die mit dem mittleren Abschnitt in einer Richtung, die transversal zur Länge der Schlinge (16) ist, gekoppelt sind.

16. System nach Anspruch 1, ferner mit einer Messvorrichtung zum Messen des Winkels zwischen dem Anus und dem Rektum (37), wobei die Messvorrichtung einen proximalen Ring und einen distalen Ring hat, die mittels eines Gelenks betriebsfähig verbunden sind, wobei der Winkel auf einer an dem proximalen Ring angebrachten visuellen Skala angezeigt wird.

17. System nach Anspruch 16, wobei die Messvorrichtung konfiguriert ist, an einem Finger angeordnet zu werden.

18. System nach Anspruch 1, wobei jeder Arm eine entfernbare Hülle aufweist, um sein Hindurchführen durch ein Hüftbeinloch zu erleichtern.

19. System nach Anspruch 1, wobei die Schlinge (16) ein Material aus der folgenden Gruppe aufweist: ein synthetisches Material, ein natürliches xenogenes Transplantatmaterial, ein allogenes Transplantatmaterial und Kombinationen davon.

20. System nach Anspruch 18, wobei jeder Arm (18, 19) der Schlinge (16) aus einem elastischen Material hergestellt ist.

## Revendications

1. Système pour réduire l'incontinence anale comprenant une sangle rectale (16) implantable dans un patient et une aiguille introductrice (11) ayant une pointe d'aiguille (14) et une tige d'aiguille, ladite aiguille introductrice (11) pouvant être attachée à la sangle rectale, la sangle rectale ayant une partie centrale (20) attachée à au moins deux bras (18, 19) se prolongeant de celle-ci, **caractérisé par** : la partie centrale ayant une forme incurvée préformée qui est plus large que lesdits au moins deux bras et qui, après implantation, est structurée pour être conforme au contour externe de l'anus, du rectum, d'un angle non rectal et/ou des muscles releveurs de l'anus dans ledit patient.

2. Système selon la revendication 1, dans lequel ladite aiguille introductrice (11) comprend en outre une gaine creuse (31) recouvrant ladite aiguille introductrice.

3. Système selon la revendication 1, dans lequel ladite pointe d'aiguille (14) est retirable par dévissage de ladite tige d'aiguille.

4. Système selon la revendication 3, comprenant en outre une vis de type connecteur mâle (36) ayant une extrémité filetée et une extrémité non filetée, ladite extrémité non filetée étant couplée auxdits bras (18, 19).

5. Système selon la revendication 4, dans lequel ladite vis de type connecteur mâle (36) est structurée pour se coupler par vissage à ladite tige d'aiguille lors du retrait de ladite pointe d'aiguille.

6. Système selon la revendication 1, dans lequel ladite pointe d'aiguille comprend une mâchoire pour attraper lesdits bras.

7. Système selon la revendication 2, dans lequel lesdits bras (18, 19) comprennent en outre une suture couplée à ceux-ci.

8. Système selon la revendication 7, dans lequel ladite gaine comprend un mécanisme de ressort pour déployer la pointe d'aiguille (14).

9. Système selon la revendication 7 ou 8, dans lequel ladite pointe d'aiguille (14) comprend une encoche pour un couplage de manière fonctionnelle à ladite suture.

10. Système selon la revendication 1, dans lequel la partie centrale (20) comprend un sac gonflable de manière ajustable.

11. Système selon la revendication 10, dans lequel ledit sac gonflable de manière ajustable comprend en outre un orifice accessible par voie sous-cutanée positionné sur un fond de la partie centrale pour l'introduction et le retrait d'un liquide.

12. Système selon la revendication 10, dans lequel ledit sac gonflable de manière ajustable comprend en outre un tube de connecteur (42) ayant des première et seconde extrémités, ledit tube de connecteur étant couplé audit sac au niveau de la première extrémité et ayant un orifice couplé à celui-ci au niveau de la seconde extrémité pour introduire un liquide dans et retirer un liquide dudit sac.

13. Système selon la revendication 1, dans lequel lesdits bras (18, 19) comprennent des dentelures sur ceux-ci pour maintenir ladite sangle (16) en position après son implantation.

14. Système selon la revendication 1, comprenant en outre des dispositifs de fixation pour attacher lesdits bras (18, 19) à l'os iliaque, lesdits dispositifs de fixation étant choisis dans le groupe constitué par des ancrages osseux, un matériau de suture et des combinaisons de ceux-ci.

15. Système selon la revendication 1, dans lequel ladite partie centrale (20) comprend en outre des éléments rigides, semi-rigides ou souples couplés à ladite partie centrale dans un sens transversal à la longueur de la sangle (16).

16. Système selon la revendication 1, comprenant en outre un dispositif de mesure pour mesurer l'angle entre l'anus et le rectum (37), ledit dispositif de mesure ayant un anneau proximal et un anneau distal reliés de manière fonctionnelle par une jointure dans lequel ledit angle est affiché sur une échelle visuelle couplée audit anneau proximal.

17. Système selon la revendication 16, dans lequel ledit dispositif de mesure est structuré pour s'adapter sur un doigt.

18. Système selon la revendication 1, dans lequel chaque bras comprend une gaine pouvant être éliminée pour faciliter le passage à travers un trou ischio-pubien.

19. Système selon la revendication 1, dans lequel la sangle (16) comprend un matériau choisi dans le groupe constitué par un matériau synthétique, un matériau naturel pour xénogreffe, un matériau pour allogreffe et des combinaisons de ceux-ci.

20. Système selon la revendication 18, dans lequel chaque bras (18, 19) de la sangle (16) est constitué de matériau élastique.
